# EUROPEAN PATENT APPLICATION

(11) **EP 4 748 292 A1**
(43) Date of publication of application: **27.05.2026**
(21) Application number: 25213682.5
(22) Date of filing: 05.11.2025
(51) Int. Cl.: A61B 1/00, A61B 1/005, A61B 1/018

(54) **SHAFT, SURGICAL INSTRUMENT AND METHOD FOR ASSEMBLING A WORKING CHANNEL AND/OR AN OPTICAL IMAGE DEVICE TO A SHAFT**

(30) Priority: 21.11.2024 DE 102024134352
(71) Applicant: Karl Storz SE & Co. KG, 78532 Tuttlingen (DE)
(72) Inventor: Polluks, Indrek-Toomas, 78532 Tuttlingen (DE)

(57) **Abstract**

The present invention provides a shaft (1) for a surgical instrument, in particular for a flexible endoscopic instrument, comprising: an inner shaft structure layer (2) having a distal end (3) and a proximal end (4), wherein the inner shaft structure layer (2) comprises a through-opening (5) for receiving a working channel and/or an optical image device, wherein the through-opening (5) extends axially from the proximal end (4) to the distal end (3), and wherein the inner shaft structure layer (2) comprises a slot (6) for laterally opening the inner shaft structure layer (2), wherein the slot (6) is arranged at least sectionwise between the proximal end (4) and the distal end (3). Further the present invention provides a surgical instrument (100) comprising such a shaft (1) as well as a method for assembling a working channel and/or an optical image device to a shaft (1) for a surgical instrument.

## Description

### TECHNICAL FIELD

The present invention pertains to a shaft for a surgical instrument, in particular for a flexible endoscopic instrument, as well as a surgical instrument, in particular flexible endoscopic instrument, with such a shaft. Furthermore, the present invention pertains to a method for assembling a working channel and/or an optical image device to a shaft for a surgical instrument, in particular to such a shaft.

### BACKGROUND

Surgical tube shaft instruments such as flexible endoscopic instruments are used for a variety of applications in medicine and technology. Such flexible endoscopic instruments comprise a flexible elongated shaft which is suitable for insertion into a cavity, such as an internal body cavity or a cavity of a technical object. As a rule, an endoscopic instrument lens is arranged at the tip of the endoscopic instrument shaft to generate an image of a scene in the observed cavity. To record and transmit the endoscopic image from the distal (i.e. far from the observer) to the proximal (i.e. close to the observer) end region of the endoscopic instrument, an ordered bundle of optical fibers running inside the shaft can be provided, for example, or an electronic image sensor, such as a CCD chip, which is arranged in the region of the distal end of the shaft and whose signals are transmitted to the proximal end region via electrical lines running inside the shaft. Since there is usually not enough light in the observed cavity, a light guide system can also be arranged inside the shaft to transport light to the distal end of the endoscopic instrument, where it is used to illuminate the cavity. Further, the endoscopic instrument shaft may include one or more working channels for passing endoscopic working instruments from the proximal to the distal end portion of the shaft for performing manipulations within the cavity.

Surgical tube shaft instruments are also known which comprise a flexible elongated shaft which is also suitable for insertion into a cavity, such as an internal body cavity or a cavity of a technical object. Such a flexible endoscopic instrument can be used to carry out manipulations in the cavity and for this purpose can be designed, for example, as a grasping instrument for grasping and manipulating tissue or objects in the cavity inside the body or in the cavity of a technical object. For this purpose, a tool is arranged at the distal end of the shaft, which can be operated from the proximal end of the shaft via a transmission means running inside the shaft. Such a flexible endoscopic instrument usually does not have its own optics for recording an endoscopic image, but can be used in particular together with a flexible endoscopic instrument.

It is often desirable to be able to angle the distal end of the shaft, i.e. the tip of the endoscope or endoscopic instrument, in order to facilitate the insertion of the endoscope or endoscopic instrument through a non-flexible endoscopic instrument through a non-rectilinear channel, to be able to move the tip within a cavity in a lateral direction and to be able to change the viewing direction of an optical system arranged in the endoscopic instrument tip or the working direction of a tool arranged at the tip of the endoscopic instrument. For this purpose, the shaft has a controllable section, in particular a controllable end section, which can be actively angled by a desired amount in a desired direction and can be controlled for this purpose from the proximal end of the endoscopic instrument. The shaft for a flexible endoscopic instrument need not itself have a tool and a transmission means, but can for example comprise a working channel into which a flexible endoscopic working instrument which cannot be actively angled and which has such a tool can be inserted up to the distal end of the shaft and possibly beyond, so that the flexible working instrument can be angled with the aid of the shaft.

The flexible shaft is usually part of the flexible endoscope, which transfers the torque and force from a handle to a distal section. Since the flexible shaft is hollow it protects also the internal components, like the working channel, light/imager cables, springs and/or push-pull wires.

In document US 2021/153728 A1 endoscopic devices comprising one or more working channels and methods for producing an endoscopic device with improved working channels are described.

However, loading of components of the flexible endoscopic instrument to the working shaft is time consuming work and so far there is no automation solution.

### DISCLOSURE OF THE INVENTION

It is an object of the present invention to provide a shaft, which can simplify an assembling process of a working channel for a surgical instrument.

According to the invention, this problem is solved by a shaft for a surgical instrument with the features of claim 1, by a surgical instrument with the features of claim 12 and/or by a method for assembling a working channel and/or an optical image device to a shaft for a surgical instrument with the features of claim 13.

According to a first aspect of the invention, a shaft for a surgical instrument, in particular for a flexible endoscopic instrument, is provided. The shaft comprises an inner shaft structure layer having a distal end and a proximal end, wherein the inner shaft structure layer comprises a through-opening for receiving a working channel and/or an optical image device. The through-opening extends axially from the proximal end to the distal end. The inner shaft structure layer comprises a slot for laterally opening the inner shaft structure layer, wherein the slot is arranged at least sectionwise between the proximal end and the distal end.

According to a second aspect of the invention, a surgical instrument, in particular flexible endoscopic instrument, is provided. The surgical instrument comprises a shaft according to the first aspect of the invention arranged at a distal end of the surgical instrument.

According to a third aspect of the invention, a method for assembling a working channel and/or an optical image device to a shaft for a surgical instrument, in particular to a shaft according to the first aspect of the invention, is provided. The method comprises the steps of:
Providing a shaft comprising an inner shaft structure layer having a distal end and a proximal end, wherein the inner shaft structure layer comprises a through-opening for receiving a working channel and/or an optical image device, wherein the through-opening extends axially from the proximal end to the distal end, and wherein the inner shaft structure layer comprises a slot for laterally opening the inner shaft structure layer, wherein the slot is arranged at least sectionwise between the proximal end and the distal end.
Loading the working channel through the slot into the through-opening.

A fundamental concept of the invention is to provide a flexible endoscope working vertebrae or shaft, respectively, including the inner shaft structure layer or internal profile, respectively, for all main components of the surgical instrument, in particular flexible endoscopic instrument. The inner shaft structure layer in particular accommodates steering wires, a working channel and an optical image device. The inner shaft structure layer or internal profile, respectively, comprises the slot respectively side open slot. For example, the inner shaft structure layer can be configured as a carcass structure. The distal end of the shaft corresponds to the distal end of the surgical instrument. Furthermore, the methods disclose a way of assembling the vertebrae to the endoscopic instrument.

A particular advantage in the solution according to an aspect of the invention is that loading of the components of the shaft can be at least partly automated.

A further advantage of the present invention is that an easier and faster assembly of the working channel and/or the optical image device can be provided.

The inner shaft structure layer can be based on an extruded profile, for example. That means, the inner shaft structure layer can be manufactured by an extrusion process. For example, the working channel can be loaded into the through-opening by pushing open the slot.

The working channel can be inserted into a tubular shaft of the surgical instrument. The working channel is mounted or guided at least at an end, in particular an proximal end, of a rod in the surgical instrument. In particular, the rod is mounted or guided in the tubular shaft in such a way that the working channel cannot move or rotate axially relative to the tube shaft.

If the surgical instrument is to be operated by a user such as a surgeon, the surgical instrument comprises a handle as a grip for the user. The operating interface then transmits a force or torque, which is applied by the user to the handle via a suitable actuation (e.g. movable handle leg), to a force transmission element for example.

If the surgical instrument is to be connected to a robot, the surgical instrument comprises a corresponding connection interface for the robot, which can apply an axial force or torque to the force transmission element via the operating interface.

Advantageous embodiments and further developments emerge from the description with reference to the figures.

According to some embodiments of the invention, the inner shaft structure layer has an inner surface and an outer surface facing away from the inner surface, wherein the slot extends radially from the outer surface to the inner surface. Thus, the slot splits the inner shaft structure layer through its complete thickness.

According to some further embodiments of the invention, the shaft further comprises an outer shaft layer, which is disposed on the outer surface of the inner shaft structure layer. Hence, the outer shaft layer can support the inner shaft structure layer. For example, the outer shaft layer may comprise strengthening elements for strengthening the shaft. The outer shaft layer can be configured as a coating that partly encloses the inner shaft structure layer.

According to some further embodiments of the invention, the inner shaft structure layer and the outer shaft layer are bonded together materially.

According to some further embodiments of the invention, the inner shaft structure layer and the outer shaft layer have a press fit.

Optionally, the inner shaft structure layer and the outer shaft layer can have a press fit and be bonded together materially.

According to some further embodiments of the invention, the outer shaft layer comprises a meshed cover, metal or a liquid crystal polymer. Additionally, the outer shaft layer can be meshed together with a biocompatible material like polyurethane, thermoplastic elastomer or impregnated pellethane. In particular, the outer shaft layer can comprise a meshed polymer area, for example in combination with a pure polymer area.

According to some further embodiments of the invention, the shaft further comprises an intermediate layer, which is arranged radially between the inner shaft structure layer and the outer shaft layer. For example, the intermediate layer is configured as a heat activated film such that it materially bonds to the inner shaft structure layer and to the outer shaft layer. Hence, glue rings on the distal end can be avoided, in particular in case the heat activated film is applied at the distal end.

According to some further embodiments of the invention, the slot is arranged in a section of the inner shaft structure layer that is configured to be articulatable at least in one plain. Thus, the section builds a flexible shaft section. In embodiments in which the shaft comprises the articulatable section and the outer shaft layer with a mesh, the mesh can be omitted in the articulatable section. In particular, the outer shaft layer can comprise a meshed polymer area for the shaft apart from the section and a pure polymer area for the section.

Optionally, the section comprises a plurality of shaft sections arranged in series, wherein the slot extends axially through the plurality of shaft sections. The plurality of shaft sections arranged in series correspond to an active bending section of the surgical instrument, in particular flexible endoscopic instrument.

According to some further embodiments of the invention, the inner shaft structure layer has a recess, which penetrates the inner shaft structure layer on two opposing sides with respect to the slot in an alignment. Thus, any longitudinal body, for example a pin, can be put into the recess for securing the slot against opening.

According to some further embodiments of the invention, the slot forms a bay with respect to the axial extension of the inner shaft structure layer. The bay can be shaped as a U, as a V, as a hammer, as a peninsula or the like.

According to some further embodiments of the invention, the slot is arranged at two opposing lateral sides of the inner shaft structure layer. Hence, opening the inner shaft structure layer can be simplified.

Optionally, the inner shaft structure layer can be elastically deformable at least in an area of the slot.

Optionally, the surgical instrument may further comprise a working channel for communicating fluids or medical tools from an operating interface to the distal end of the shaft and two steering wires extending from the operating interface through the inner shaft structure layer for articulating a section of the inner shaft structure layer.

The operating interface and/or the surgical instrument can be made of a plastic material. For example, the surgical instrument can be completely made of a plastic material, in particular when the surgical instrument is intended to use only once.

According to some further embodiments of the invention, the method further comprises a step of disposing an outer shaft layer on the inner shaft structure layer after the working channel has been loaded. In particular, the outer shaft layer can be pulled over the inner shaft structure layer.

Additionally or alternatively, an intermediate layer can be disposed on the inner shaft structure layer after the working channel has been loaded. In this case, the outer shaft layer can be disposed on the intermediate layer. Furthermore, the outer shaft layer can be manufactured by an extrusion process, for example.

According to some further embodiments of the invention, the step of disposing the outer shaft layer includes expanding the outer shaft layer by pressurized air such that the outer shaft layer is pulled over the inner shaft structure layer or the intermediate layer, respectively. Thus, the outer shaft layer can be compressed when stopping the pressurized air such that the inner shaft structure layer and the outer shaft layer have a press fit.

Optionally, the method can further comprise a step of heating the outer shaft layer such that the outer shaft layer materially bonds to the inner shaft structure layer. For example, the heating can be applied by infrared heating.

The plurality of shaft sections can be connected by an integrated connecting bar, which connects adjacent shaft sections such that they are articulatable in one plain. For example, each shaft section can be configured as a vertebral member, which is coupled to the adjacent vertebral member such that pivoting of the vertebral member relative to the adjacent vertebral member in a radial axis is provided. For example, the inner shaft structure layer can comprise a circular or oval shape. Thereby, the inner shaft structure layer can have a constant diameter.

Moreover, the surgical instrument can further comprise a working channel for communicating fluids or medical tools from the operating interface to the distal end of the shaft and two steering wires extending from the operating interface through the through-opening for articulating the shaft.

In other words, the distal tip with vertebrae subassembly can be loaded to the shaft through the slot respectively side opening slot and the outer shaft cover can be pulled over the structure respectively inner shaft structure layer. The compressed air can be used to expand the outer shaft cover. The outer shaft cover can be bonded to internal shaft layer respectively inner shaft structure layer using heating/IR heating. The result might be a supported loaded shaft that can transmit force and torque.

The above embodiments and further developments can be combined with one another arbitrarily, as far as appropriate. Further possible configurations, developments and implementations of the invention are also combinations of features of the invention described above or below for the exemplary embodiments that are not explicitly cited. In particular, a person skilled in the art will also add individual aspects as improvements or additions to the respective basic form of the present invention.

### BRIEF DESCRIPTION OF THE DRAWINGS

The present invention is explained more specifically below on the basis of the exemplary embodiments indicated in the schematic figures, in which:
Fig. 1 shows a handheld surgical instrument, in particular a steerable flexible endoscopic instrument, in an overall view according to an embodiment of the invention;
Fig. 2 shows a perspective view of a shaft for a flexible endoscopic instrument according to a further embodiment of the invention;
Fig. 3 shows perspective view of a shaft for a flexible endoscopic instrument according to a further embodiment of the invention;
Fig. 4 shows a perspective view of the shaft of Fig. 2 or 3 comprising an outer shaft layer according to a further embodiment of the invention;
Fig. 5 shows each a side view and a front view of a cut section of two shafts comprising an intermediate layer and an outer layer according to a further embodiment of the invention;
Fig. 6 shows a detail view of a shaft section of the shaft of Fig. 2;
Fig. 7 shows a cut view of a shaft according to a further embodiment of the invention;
Fig. 8 shows a cut view of a shaft according to a further embodiment of the invention; and
Fig. 9 shows a schematic flow chart of a method for assembling a working channel and/or an optical image device to a shaft for a surgical instrument according to a further embodiment of the invention.

The accompanying figures are intended to convey a further understanding of the embodiments of the invention. They illustrate embodiments and are used in conjunction with the description to explain principles and concepts of the invention. Other embodiments and many of the cited advantages emerge in light of the drawings. The elements of the drawings are not necessarily shown to scale in relation to one another. Direction-indicating terminology such as for example "at the top", "at the bottom", "on the left", "on the right", "above", "below", "horizontally", "vertically", "at the front", "at the rear" and similar statements are merely used for explanatory purposes and do not serve to restrict the generality to specific configurations as shown in the figures.

In the figures of the drawing, elements, features and components that are the same, have the same function and have the same effect are each provided with the same reference signs - unless explained otherwise.

### DETAILED DESCRIPTION OF DRAWINGS

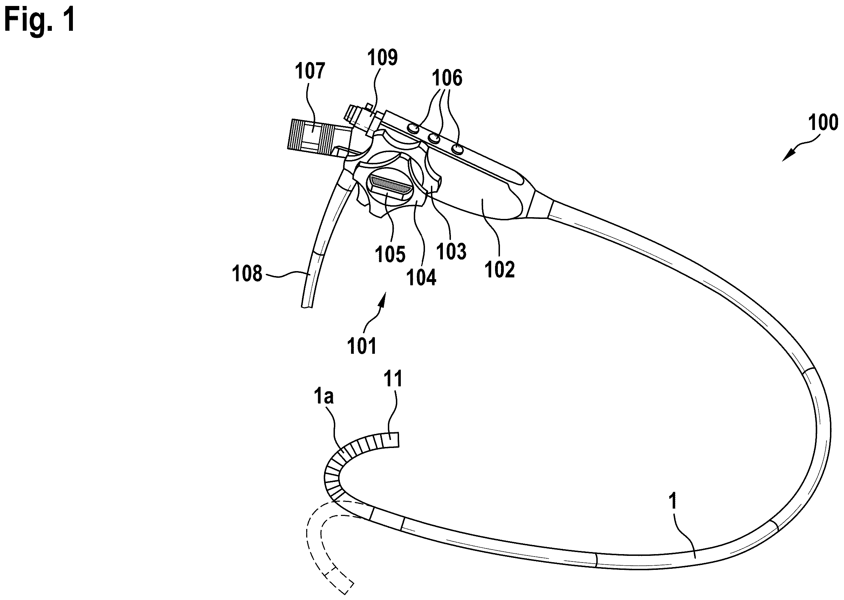

Fig. 1 shows an embodiment of a handheld surgical instrument 100, in particular of a steerable flexible endoscopic instrument 100, in an overall view.

As shown schematically in Fig. 1, the flexible endoscopic instrument 100 typically comprises a handpiece 101 and a shaft 1, the handpiece 101 being attached to a proximal end of the shaft 1. The handpiece 101 can have an outer housing 102 made of a plastic and/or metallic material. On a lower side of the housing 102 a first hand wheel 103 and a second hand wheel 104 are arranged for controlling a deflection of a steerable section 1a of the shaft 1, as is described below. Typically the first and the second hand wheel 103, 104 are arranged coaxially, and at an exterior side of the second hand wheel 104 a knob 105 for controlling a deflection brake relating to the second hand wheel 104 is provided. Further, the handpiece 101 may exhibit a multiplicity of control buttons 106 for controlling various functions of the flexible endoscopic instrument 100, such as for controlling the imaging and/or illumination system and/or irrigation and suction pumps, for example. The handpiece 101 may be connectable to an external video unit or a video monitor via connector 107 and to an external light source via light cable 108. Moreover, an instrument port 109 may be provided for inserting endoscopic instruments to be advanced through one or more respective channels to a distal end of the shaft 1 for manipulating tissue or other objects within a cavity into which the shaft 1 can be inserted.

At its distal end the shaft 1 comprises a steerable section 1a. The steerable section 1a may form a distal end section of the shaft 1 or, as shown in Fig. 1, may carry a distal end cap 11, which may accommodate an imaging optics and an electronic image sensor for providing an endoscopic image of a cavity into which the shaft 1 is inserted. The image signal generated by the image sensor may be transmitted via electric cables extending through the shaft 1 and the handpiece 101 to the connector 107 for being processed and displayed by an external video unit. The shaft 1 in total is flexible to an extent to be advanced through an endoscopic access or a hollow organ towards a cavity to be observed, being capable of adapting to a curved shape of the access or the organ. The steerable section 1a, on the other hand, is capable of being flexed actively by turning the hand wheels 103, 104. To this end the steerable section 1a comprises an inner structure of a plurality of shaft sections, thus being deflectable or articulatable in one or more planes. The plurality of shaft sections can be covered by a flexible tube to form a smooth outer surface, the shaft 1 in total having a uniform cross-sectional shape and diameter.

For controlling the deflection of the plurality of shaft sections two counteracting control wires (not shown in Fig. 1) are provided extending on opposite sides within the plurality of shaft sections, by a longitudinal movement of which the steerable section 1a can be bent to one or the other side, as indicated symbolically in Fig. 1. The control wires extend along the shaft 1 and are connected at their proximal ends to a deflection control mechanism arranged within the handpiece 101, which can be operated by a user by turning the hand wheels 103, 104 for deflecting the steerable section 1a. In the exemplary embodiment shown, the steerable section can be bent or articulated within a first plane that corresponds to the plane of the drawing, a deflection angle being controllable by rotating the first hand wheel 103. Further, the steerable section 1a can be bent in a second plane perpendicular to the first plane and perpendicular to the plane of the drawing, a corresponding deflection angle being controllable by rotating the second hand wheel 104.

The shaft 1 exemplarily comprises an inner shaft structure layer having a distal end and a proximal end, wherein the inner shaft structure layer comprises a through-opening for receiving a working channel and/or an optical image device. The through-opening extends axially from the proximal end to the distal end. Furthermore, the inner shaft structure layer comprises a slot for laterally opening the inner shaft structure layer, wherein the slot is arranged at least sectionwise between the proximal end and the distal end. For example, the slot substantially extends axially at least sectionwise between the proximal end and the distal end.

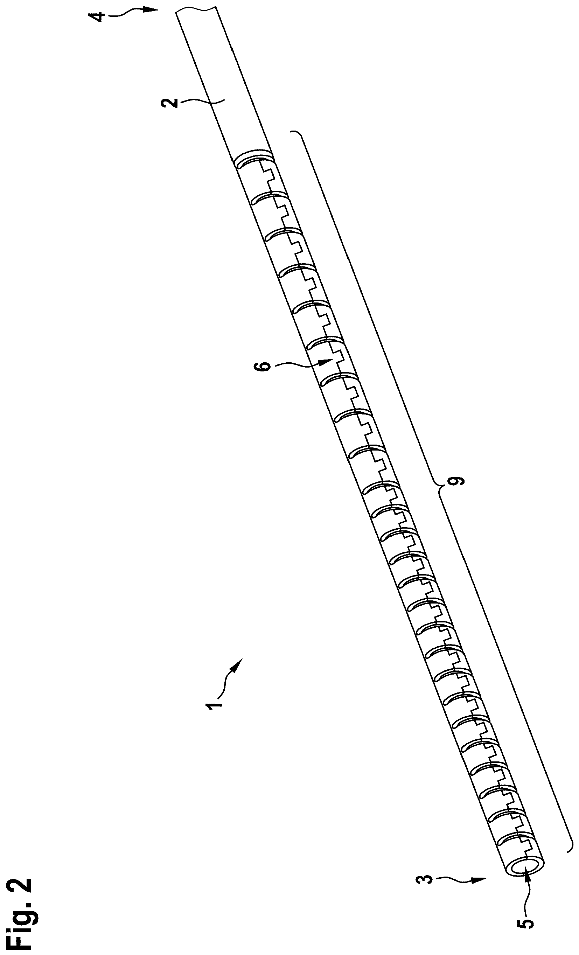

Fig. 2 shows a perspective view of a shaft 1 for a flexible endoscopic instrument.

The flexible endoscopic instrument (not shown) can be exemplarily configured as a handheld endoscopic instrument comprising an endoscopic instrument handle for manual operation by a user, as it is exemplarily illustrated in Fig. 1. Alternatively, the endoscopic instrument handle can be configured as a corresponding interface for a robot for guidance by a robot. The flexible endoscopic instrument comprises the shaft 1, which is arranged at a distal end of the flexible endoscopic instrument. The flexible endoscopic instrument may further comprise an operating interface, wherein the shaft 1 is coupled to the operating interface. The shaft 1 and the working channel can be configured as a round tube. The working channel is configured for communicating fluids or medical tools. Furthermore, the shaft 1 can be arranged concentric in the operating interface.

The shaft 1 comprises an inner shaft structure layer 2 having a distal end 3 and a proximal end 4. The inner shaft structure layer 2 comprises a through-opening 5 for receiving a working channel or an optical image device or both. The through-opening 5 extends axially from the proximal end 4 to the distal end 3.

The inner shaft structure layer 2 comprises a slot 6 for laterally opening the inner shaft structure layer 2, wherein the slot 6 is arranged at least sectionwise between the proximal end 4 and the distal end 3. Optionally, the inner shaft structure layer 2 can be elastically deformable at least in an area of the slot 6.

In Fig. 2, the slot 6 is exemplarily arranged in a section 9 of the inner shaft structure layer 2 that is configured to be articulatable at least in one plain. Thus, the section 9 builds a flexible shaft section.

Further, the section 9 can comprise a plurality of shaft sections, which are arranged in series between the distal end 3 and the proximal end 4. The plurality of shaft sections can be articulatable in one plain and comprises the through-opening 5. The plurality of shaft sections can be made of a single type of plastic material, which is elastically deformable. Each shaft section may be configured as a vertebral member, which is coupled to the adjacent vertebral member such that pivoting of the vertebral member relative to the adjacent vertebral member in a radial axis is provided. Here, the plurality of shaft sections exemplarily comprises a circular shape for forming a circular shaped shaft 1. Thereby, the shaft 1 has a constant diameter. But the shape of the shaft 1 is not limited to this and can also have an oval or similar shape. For example, the shaft 1 and the through-opening 5 can have the same shape with a different diameter or width. Alternatively, the shaft 1 and the through-opening 5 can have different shapes.

For example, the slot 6 can substantially extend axially at least sectionwise between the proximal end and the distal end in the section 9. In particular, the slot 6 can extend axially through the plurality of shaft sections. The plurality of shaft sections arranged in series correspond to an active bending section of the surgical instrument, in particular flexible endoscopic instrument.

Furthermore, the slot 6 can form a bay with respect to the axial extension of the inner shaft structure layer 2. For example, the slot 6 forms a bay in each shaft section in the section 9. The bay can be shaped as a U, as a V, as a hammer, as a peninsula or the like. Here, the bay is exemplarily shaped as a rectangular peninsula.

The through-opening 5 is configured for receiving the working channel or the optical image device or both. In particular, the through-opening 5 can further be configured for receiving two steering wires. Thereby, the through-opening 5 can extend through the plurality of shaft sections axially. The two steering wires can extend here from an operating interface of the flexible endoscopic instrument to articulate the shaft 1, in particular the section 9.

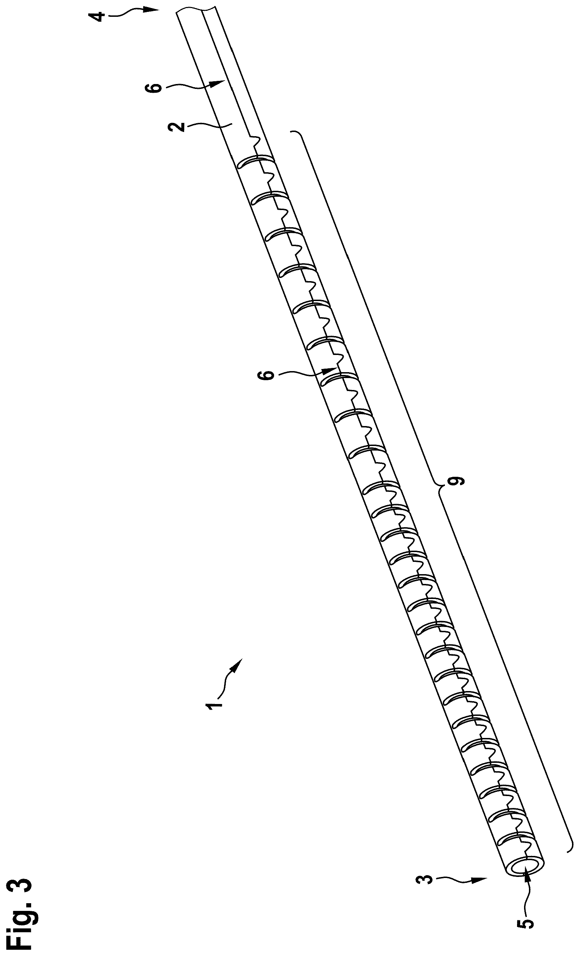

Fig. 3 shows perspective view of a shaft 1 for a flexible endoscopic instrument according to a further embodiment of the invention.

The shaft 1 of Fig. 3 substantially comprises the same features as the shaft 1 of Fig. 2, but differs in that the slot 6 extends here between the distal end 3 and the proximal end 4, for example, that means not only in the section 9.

Furthermore, the shape of the bay of the slot 6 exemplarily forms a V or a V-like peninsula.

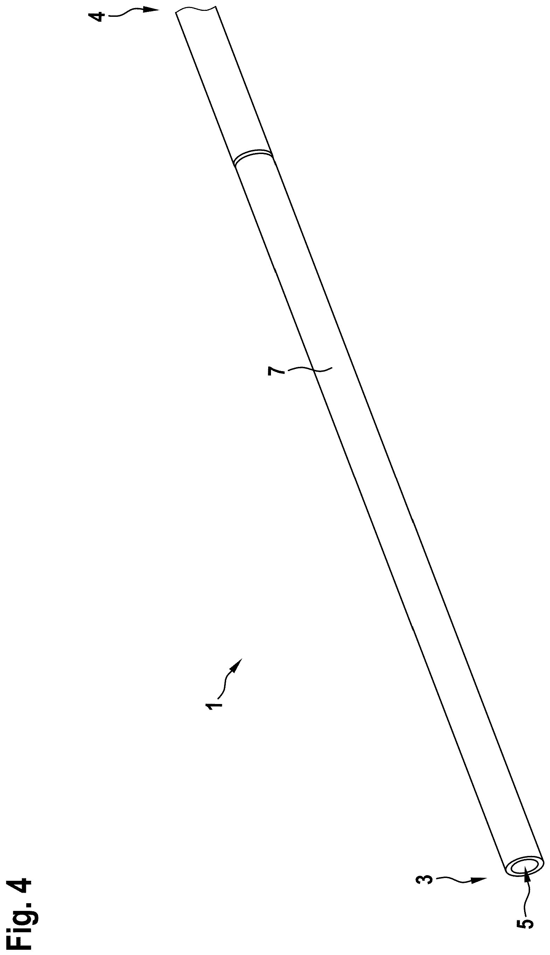

Fig. 4 shows a perspective view of the shaft 1 of Fig. 2 or 3 comprising an outer shaft layer 7 according to a further embodiment of the invention.

For example, the inner shaft structure layer 2 has an inner surface and an outer surface facing away from the inner surface. The slot 6 can extend radially from the outer surface to the inner surface. Thus, the slot 6 can split the inner shaft structure layer 2 through its complete thickness.

The outer shaft layer 7 can be disposed on the outer surface of the inner shaft structure layer 2. Hence, the outer shaft layer 7 can support the inner shaft structure layer. For example, the outer shaft layer 7 may comprise strengthening elements (not shown) for strengthening the shaft 1.

For example, the inner shaft structure layer 2 and the outer shaft layer 7 can be bonded together materially. Alternatively or additionally, the inner shaft structure layer 2 and the outer shaft layer 7 can have a press fit. In particular, the inner shaft structure layer 2 and the outer shaft layer 7 can have a press fit and be bonded together materially.

Furthermore, the outer shaft layer 7 may comprise a meshed cover, metal or a liquid crystal polymer. Additionally, the outer shaft layer 7 can be meshed together with a biocompatible material like polyurethane, thermoplastic elastomer or impregnated pellethane. In particular, the outer shaft layer 7 can comprise a meshed polymer area for the shaft 1 apart from the section 9, so in the area towards the proximal end 4, and a pure polymer area for the section 9.

Optionally, the slot 6 can be arranged at two opposing lateral sides of the inner shaft structure layer 2.

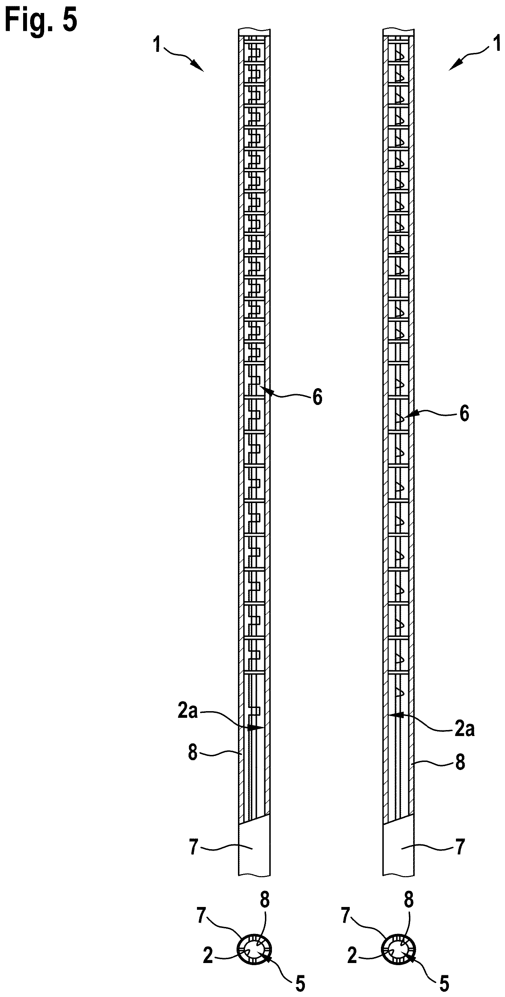

Fig. 5 shows each a side view and a front view of a cut section of two shafts 1 comprising an intermediate layer 8 and an outer layer 7 according to a further embodiment of the invention. Specifically, the front views located below the side views of the two shafts 1 correspond to the above located shafts 1 illustrated in side view.

The shaft 1 comprises an inner shaft structure layer 2 having a distal end 3 and a proximal end 4. The inner shaft structure layer 2 comprises a through-opening 5 for receiving a working channel or an optical image device or both. The through-opening 5 extends axially from the proximal end 4 to the distal end 3.

The inner shaft structure layer 2 comprises a slot 6 for laterally opening the inner shaft structure layer 2, wherein the slot 6 is arranged at least sectionwise between the proximal end 4 and the distal end 3. Optionally, the inner shaft structure layer 2 can be elastically deformable at least in an area of the slot 6.

For example, the inner shaft structure layer 2 has an inner surface 2a and an outer surface 2b facing away from the inner surface 2a, wherein the slot 6 can extend radially from the outer surface 2b to the inner surface 2a.

Furthermore, the shaft 1 can comprise the intermediate layer 8, which is arranged radially between the inner shaft structure layer 2 and the outer shaft layer 7. For example, the intermediate layer 8 can be configured as a heat activated film such that it materially bonds to the inner shaft structure layer 2 and to the outer shaft layer 7. Hence, glue rings on the distal end 3 can be avoided, in particular in case the heat activated film is applied at the distal end.

For example, the slot 6 can substantially extend axially at least sectionwise between the proximal end and the distal end in the section 9. Furthermore, the slot 6 can form a bay with respect to the axial extension of the inner shaft structure layer 2. The bay can be shaped as a U, as a V, as a hammer, as a peninsula or the like.

Both illustrated shafts 1 can have the same features, in particular as described above, apart from the slot 6, which might be arranged differently. Furthermore, the slot 6 can form a different shape of the bay when comparing the right shaft 1 with the left shaft 1. Here, the bay of the left shaft 1 is exemplarily shaped as a rectangular peninsula. The bay of the right shaft 1 is exemplarily shaped as a V or U, for example.

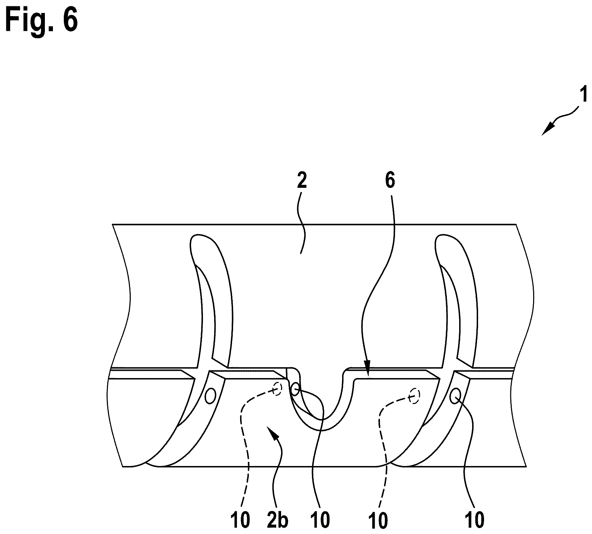

Fig. 6 shows a detail view of a shaft section of the shaft 1 of Fig. 2.

For example, the inner shaft structure layer 2 has an inner surface and an outer surface 2b facing away from the inner surface, wherein the slot 6 can extend radially from the outer surface 2b to the inner surface.

In Fig. 6, the inner shaft structure layer 2 exemplarily has a recess 10, which penetrates the inner shaft structure layer 2 on two opposing sides with respect to the slot 6 in an alignment. Thus, any longitudinal body, for example a pin, can be put into the recess 10 for securing the slot 6 against opening.

Furthermore, the slot 6 can form a bay with respect to the axial extension of the inner shaft structure layer 2. In particular, the recess can be arranged in the bay. Optionally, the recess 10 can extend parallel to the through-opening 5. Moreover, the recess 10 can extend through the plurality of shaft sections.

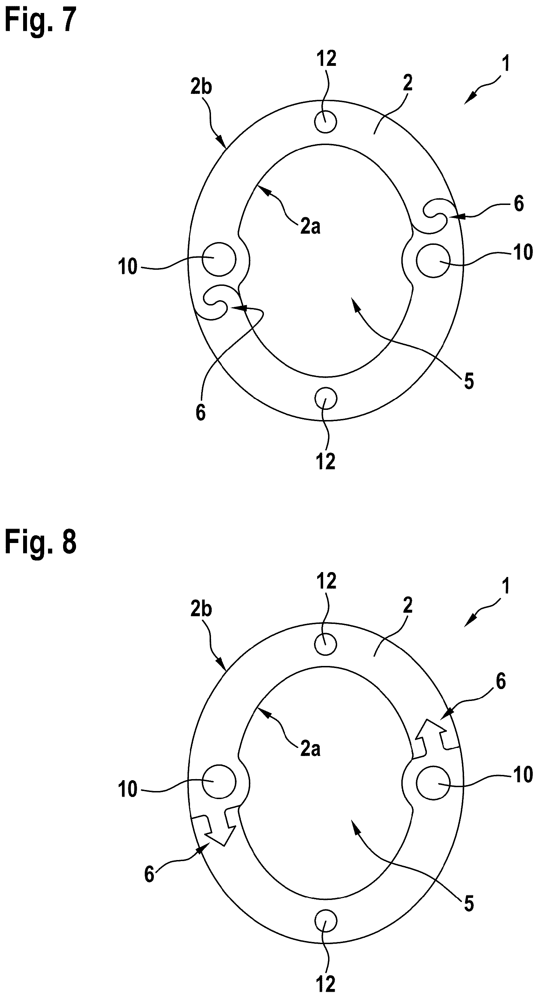

Fig. 7 shows a cut view of a shaft 1 according to a further embodiment of the invention.

Here, the inner shaft structure layer 2 has an inner surface 2a and an outer surface 2b facing away from the inner surface 2a, wherein the slot 6 can extend radially from the outer surface 2b to the inner surface 2a.

In Fig. 7, the inner shaft structure layer 2 exemplarily has a recess 10, which penetrates the inner shaft structure layer 2 on two opposing sides with respect to the slot 6 in an alignment. Thus, any longitudinal body, for example a pin, can be put into the recess 10 for securing the slot 6 against opening. Alternatively, two steering wires respectively control wires can be put into the recess 10 for articulating a steerable section of the inner shaft structure layer 2.

Furthermore, the slot 6 can form a mechanical lock like a hook. Optionally, the recess 10 can extend parallel to the through-opening 5. Moreover, the recess 10 can extend through the plurality of shaft sections. The inner shaft structure layer 2 can be manufactured by an extrusion process, in particular by an extrusion of two pieces. The two pieces can be secured by applying ultrasonic welding.

Optionally, the inner shaft structure layer 2 can comprise tunnels 12 for receiving a strengthening member. In particular, the tunnels 12 and the recesses 10 can be arranged alternating around the circumference of the inner shaft structure layer 2.

Fig. 8 shows a cut view of a shaft 1 according to a further embodiment of the invention.

Here, the inner shaft structure layer 2 has an inner surface 2a and an outer surface 2b facing away from the inner surface 2a, wherein the slot 6 can extend radially from the outer surface 2b to the inner surface 2a.

In Fig. 8, the inner shaft structure layer 2 exemplarily has a recess 10, which penetrates the inner shaft structure layer 2 on two opposing sides with respect to the slot 6 in an alignment. Thus, any longitudinal body, for example a pin, can be put into the recess 10 for securing the slot 6 against opening. Alternatively, two steering wires respectively control wires can be put into the recess 10 for articulating a steerable section of the inner shaft structure layer 2.

Furthermore, the slot 6 can form a mechanical lock like a mushroom head lock. Optionally, the recess 10 can extend parallel to the through-opening 5. Moreover, the recess 10 can extend through the plurality of shaft sections. The inner shaft structure layer 2 can be manufactured by an extrusion process, in particular by an extrusion of two pieces. The two pieces can be secured by applying ultrasonic welding.

Optionally, the inner shaft structure layer 2 can comprise tunnels 12 for receiving a strengthening member. In particular, the tunnels 12 and the recesses 10 can be arranged alternating around the circumference of the inner shaft structure layer 2.

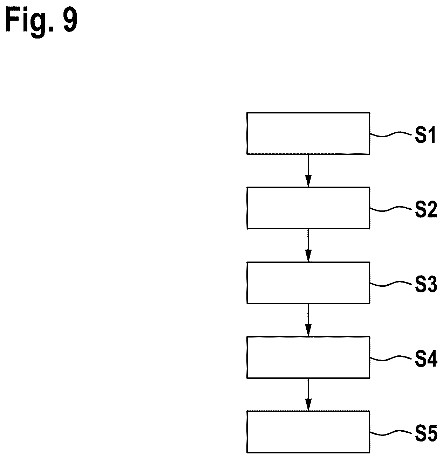

Fig. 9 shows a schematic flow chart of a method for assembling a working channel and/or an optical image device to a shaft 1 for a surgical instrument. The surgical instrument can be configured as the surgical instrument 100 of Fig. 1, for example.

The method comprises steps of providing S1 a shaft 1, loading S2 a working channel, optionally disposing S3 an intermediate layer 8, optionally disposing S4 an outer shaft layer 7 and optionally heating S5 the outer shaft layer 7.

In the step of providing S1, a shaft 1 comprising an inner shaft structure layer 2 having a distal end 3 and a proximal end 4 is provided. The inner shaft structure layer 2 comprises a through-opening 5 for receiving a working channel and/or an optical image device. The through-opening 5 extends axially from the proximal end 4 to the distal end 3. Furthermore, the inner shaft structure layer 2 comprises a slot 6 for laterally opening the inner shaft structure layer 2, wherein the slot 6 is arranged at least sectionwise between the proximal end 4 and the distal end 3. The inner shaft structure layer 2 can be based on an extruded profile, for example. That means, the inner shaft structure layer 2 can be manufactured by an extrusion process.

In the step of loading S2, the working channel is loaded through the slot 6 into the through-opening 5. For example, the working channel can be loaded into the through-opening 5 by pushing open the slot 6.

In the optional step of disposing S3, the intermediate layer 8 can be disposed S3 on the inner shaft structure layer 2 after the working channel has been loaded S2.

In the optional step of disposing S4, the outer shaft layer 7 can be disposed on the inner shaft structure layer 2 after the working channel has been loaded S2. In particular, the outer shaft layer 7 can be pulled over the inner shaft structure layer 2 or the intermediate layer when present. Furthermore, the outer shaft layer 7 can be manufactured by an extrusion process, for example.

Further, the step of disposing S4 the outer shaft layer 7 can include expanding the outer shaft layer 7 by pressurized air such that the outer shaft layer 7 is pulled over the inner shaft structure layer 2 or the intermediate layer 8, respectively. Thus, the outer shaft layer 7 can be compressed when stopping the pressurized air such that the inner shaft structure layer 2 and the outer shaft layer 7 have a press fit.

In the optional step of heating S5, the outer shaft layer 7 is heated such that the outer shaft layer 7 materially bonds to the inner shaft structure layer 2. For example, the heating S5 can be applied by infrared heating.

In the detailed description above, various features have been combined in one or more examples in order to improve the rigorousness of the illustration. However, it should be clear in this case that the above description is of merely illustrative but in no way restrictive nature. It serves to cover all alternatives, modifications and equivalents of the various features and exemplary embodiments. Many other examples will be immediately and directly clear to a person skilled in the art on the basis of his knowledge in the art in consideration of the above description.

The exemplary embodiments have been chosen and described in order to be able to present the principles underlying the invention and their application possibilities in practice in the best possible way. As a result, those skilled in the art can optimally modify and utilize the invention and its various exemplary embodiments with regard to the intended purpose of use. In the claims and the description, the terms "including" and "having" are used as neutral linguistic concepts for the corresponding terms "comprising". Furthermore, use of the terms "a", "an" and "one" shall not in principle exclude the plurality of features and components described in this way.

While at least one exemplary embodiment of the present invention(s) is disclosed herein, it should be understood that modifications, substitutions and alternatives may be apparent to one of ordinary skill in the art and can be made without departing from the scope of this disclosure. This disclosure is intended to cover any adaptations or variations of the exemplary embodiment(s). In addition, in this disclosure, the terms "comprise" or "comprising" do not exclude other elements or steps, the terms "a" or "one" do not exclude a plural number, and the term "or" means either or both. Furthermore, characteristics or steps which have been described may also be used in combination with other characteristics or steps and in any order unless the disclosure or context suggests otherwise. This disclosure hereby incorporates by reference the complete disclosure of any patent or application from which it claims benefit or priority.

### REFERENCE LIST

- 1: shaft
- 1a: steerable section
- 2: inner shaft structure layer
- 2a: inner surface
- 2b: outer surface
- 3: distal end
- 4: proximal end
- 5: through-opening
- 6: slot
- 7: outer shaft layer
- 8: intermediate layer
- 9: section of the inner shaft structure layer
- 10: recess
- 11: distal end cap
- 12: tunnel
- 100: surgical instrument
- 101: handpiece
- 102: outer housing
- 103: first hand wheel
- 104: second hand wheel
- 105: knob
- 106: control buttons
- 107: connector
- 108: light cable
- 109: instrument port

- S1: providing
- S2: loading
- S3: disposing
- S4: disposing
- S5: heating

## Claims

1. Shaft (1) for a surgical instrument (100), in particular for a flexible endoscopic instrument, comprising:
an inner shaft structure layer (2) having a distal end (3) and a proximal end (4), wherein the inner shaft structure layer (2) comprises a through-opening (5) for receiving a working channel and/or an optical image device, wherein the through-opening (5) extends axially from the proximal end (4) to the distal end (3), and wherein the inner shaft structure layer (2) comprises a slot (6) for laterally opening the inner shaft structure layer (2), wherein the slot (6) is arranged at least sectionwise between the proximal end (4) and the distal end (3).

2. Shaft (1) according to claim 1,
**characterized in that** the inner shaft structure layer (2) has an inner surface (2a) and an outer surface (2b) facing away from the inner surface (2a), wherein the slot (6) extends radially from the outer surface (2b) to the inner surface (2a).

3. Shaft (1) according to claim 2,
further comprising an outer shaft layer (7), which is disposed on the outer surface (2b) of the inner shaft structure layer (2).

4. Shaft (1) according to claim 3,
**characterized in that** the inner shaft structure layer (2) and the outer shaft layer (7) are bonded together materially.

5. Shaft (1) according to claim 3 or 4,
**characterized in that** the inner shaft structure layer (2) and the outer shaft layer (7) have a press fit.

6. Shaft (1) according to one of the claims 3 to 5,
**characterized in that** the outer shaft layer (7) comprises a meshed cover, metal or a liquid crystal polymer.

7. Shaft (1) according to one of the claims 3 to 6,
further comprising an intermediate layer (8), which is arranged radially between the inner shaft structure layer (2) and the outer shaft layer (7).

8. Shaft (1) according to one of the preceding claims,
**characterized in that** the slot (6) is arranged in a section (9) of the inner shaft structure layer (2) that is configured to be articulatable at least in one plain.

9. Shaft (1) according to one of the preceding claims,
**characterized in that** the inner shaft structure layer (2) has a recess (10), which penetrates the inner shaft structure layer (2) on two opposing sides with respect to the slot (6) in an alignment.

10. Shaft (1) according to one of the preceding claims,
**characterized in that** the slot (6) forms a bay with respect to the axial extension of the inner shaft structure layer (2).

11. Shaft (1) according to one of the preceding claims,
**characterized in that** the slot (6) is arranged at two opposing lateral sides of the inner shaft structure layer (2).

12. Surgical instrument (100), in particular flexible endoscopic instrument, comprising:
a shaft (1) according to one of the preceding claims arranged at a distal end of the surgical instrument.

13. Method for assembling a working channel and/or an optical image device to a shaft for a surgical instrument, in particular to a shaft (1) according to one of the claims 1 to 11, comprising the steps of:
providing (S1) a shaft (1) comprising an inner shaft structure layer (2) having a distal end (3) and a proximal end (4), wherein the inner shaft structure layer (2) comprises a through-opening (5) for receiving a working channel and/or an optical image device, wherein the through-opening (5) extends axially from the proximal end (4) to the distal end (3), and wherein the inner shaft structure layer (2) comprises a slot (6) for laterally opening the inner shaft structure layer (2), wherein the slot (6) is arranged at least sectionwise between the proximal end (4) and the distal end (3); and
loading (S2) the working channel through the slot (6) into the through-opening (5).

14. Method according to claim 13,
further comprising the step of disposing (S4) an outer shaft layer (7) on the inner shaft structure layer (2) after the working channel has been loaded (S2).

15. Method according to claim 14,
**characterized in that** the step of disposing (S4) the outer shaft layer includes expanding the outer shaft layer (7) by pressurized air such that the outer shaft layer (7) is pulled over the inner shaft structure layer (2).
